# EUROPÄISCHE PATENTANMELDUNG

(11) **EP 3 725 360 A1**
(43) Veröffentlichungstag der Anmeldung: **21.10.2020**
(21) Anmeldenummer: 19020291.1
(22) Anmeldetag: 16.04.2019
(51) Int. Cl.: A61N 1/05

(54) **TEMPORÄRE BIPOLARE MYOKARDIALE ELEKTRODE**

(71) Anmelder: Peter Osypka Stiftung, 79639 Grenzach-Wyhlen (DE)
(72) Erfinder: OSYPKA, Peter, 79639 Grenzach-Wyhlen (DE)

(57) **Zusammenfassung**

Die Erfindung betrifft eine temporäre bipolare myokardiale Elektrode. Die Elektrode besteht aus: zwei gegeneinander isolierte Leitern (1) und (2) mit je einem distalen und einem proximalen Ende, wobei Leiter (1) und (2) in Gebrauchsstellung aus dem Thorax nach außen ragen und mit ihrem proximalen Ende mit einem Herzschrittmacher oder EKG-Monitor verbunden werden und einem ersten proximalen indifferenten Pol (3) und einem zweiten distalen differenten Pol (4) die mit Abstand zueinander angeordnet sind und in Gebrauchsstellung im Myokard verankert sind, dadurch gekennzeichnet, dass die beiden gegeneinander isolierten Leiter (1) und (2) miteinander verbunden sind und gemeinsam bis zum proximalen indifferenten Pol (3) geführt werden, wobei Leiter (1) distal den indifferenten Pol (3) trägt und Leiter (2) isoliert außen am indifferenten Pol vorbeigeführt wird und distal den differenten Pol (4) trägt und dahinter zunächst eine später abtrennbare chirurgische Nadel aufweist.

## Beschreibung

Die Erfindung betrifft eine temporäre bipolare myokardiale Elektrode.

Temporäre Myokard-Elektroden (TME, Herzdrähte) dienen der Stimulationstherapie postoperativer Herzrhythmusstörungen. Die Elektroden werden nach herzchirurgischen Eingriffen -bei noch offenem Thorax-an der Außenseite des Vorhofs/der Vorhöfe und/oder des Ventrikels/der Ventrikel befestigt. Die elektrischen Zuleitungen werden über eine Thoraxnadel durch die Bauchdecke nach außen geführt und mit einem Herzschrittmacher oder EKG Monitor verbunden.
Die Fixierung im Myokard erfolgt über Verankerungen, nachdem der differente Elektrodenpol (Kathode) mit Hilfe einer Herznadel transmyokardial eingeführt wurde.

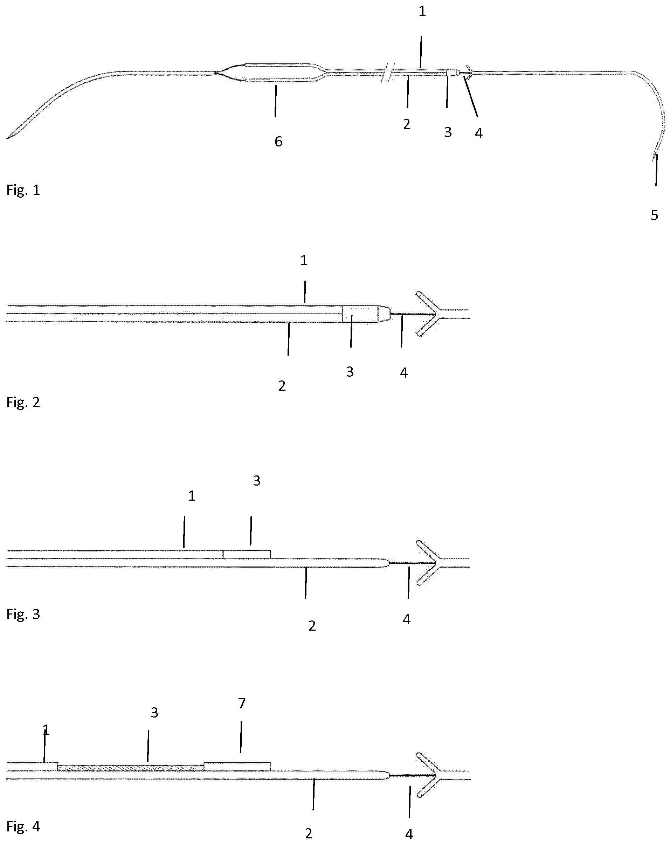

Die von Firma Osypka AG im Handel erhältlichen Myokard Elektroden sind in Fig. 1 dargestellt. Wie weiter unten dargestellt besitzt die Elektrode eine Metallhülse durch die der Leiter zum distalen Kathoden-Pol geführt wird. Da der isolierte Leiter zum distalen Pol durch die Hülse hindurch läuft kann es beim Befestigen der Metallhülse zu einer Beschädigung des weitergeführten Leiters kommen. Der Erfindung liegt die Aufgabe zugrunde eine derartige Beschädigung zu vermeiden.

Zur Lösung dieser Aufgabe wird eine bipolare myokardiale Elektrode vorgeschlagen, die aus folgenden Elementen besteht:
aus zwei gegeneinander isolierten Leitern (1) und (2) mit je einem distalen und einem proximalen Ende, wobei Leiter (1) und (2) in Gebrauchsstellung aus dem Thorax nach außen ragen und mit ihrem proximalen Ende mit einem Herzschrittmacher oder EKG-Monitor verbunden werden und
aus einem ersten proximalen indifferenten Anoden-Pol (3) und einem zweiten distalen differenten Kathoden-Pol (4), wobei Pol (3) und Pol (4) mit Abstand zueinander angeordnet sind und Pol (4) in Gebrauchsstellung im Myokard verankert ist,
dadurch gekennzeichnet, dass die beiden gegeneinander isolierten Leiter (1) und (2) miteinander verbunden sind und gemeinsam bis zum proximalen indifferenten Pol (3) geführt werden, wobei Leiter (1) distal den indifferenten Pol (3) trägt und Leiter (2) isoliert außen am indifferenten Pol vorbeigeführt wird und distal den differenten Pol (4) trägt und dahinter zunächst die später abtrennbare chirurgische Nadel aufweist.

In einer weiteren Ausführungsform ist der indifferente Anoden-Pol (3) als Metallhülse ausgebildet, die nur Leiter(1) umschließt. Die Metallhülse wird über das Ende der Isolierung von Leiter (1) geschoben und mit diesem elektrisch leitend verbunden. Dadurch wird eine Beschädigung des Leiters (2) vermieden.
Die Metallhülse ist vorzugsweise ein zylindrischer Körper. Der Zylinder kann auch konisch angespitzt sein. Die Metallhülse kann auch andere gebräuchliche Formen annehmen.
Die Metallhülse besteht aus einem gut leitenden biokompatiblen Werkstoff insbesondere aus Gold, Platin oder Platin Iridium, Edelstahl oder MP 35N.

In einer weiteren Ausführungsform ist der indifferente Anoden-Pol (3) eine vollständig blanke Litze, die distal abisoliert ist. Auch hier wird eine Beschädigung des Leiters (2) vermieden. Die blanke Litze wird durch vollständiges Entfernen der Isolierung von Leiter (1) gebildet. Die blanke Litze kann verschiedene Größen annehmen wobei ein indifferenter Pol mit variabler Oberfläche entsteht.

Die Ausführungsform mit blanker Litze hat gegenüber der Metallhülse den Vorteil, dass der Leiter im Polbereich flexibler ist.

Der differente Anker Pol (4) kann durch teilweises Entfernen der Isolierung des zu ihm führenden elektrischen Leiters (2) gebildet werden. Es können drei Teile der Isolierschicht entfernt werden, sodass es einen dreischenkligen Anker gibt, oder es können nur 2 Teile der Isolierschicht entfernt werden, sodass es einen zweischenkligen Anker gibt. Der zweischenklige Anker ist feiner und vor allem bei Kinderherzen von Vorteil. Die Isolierschicht wird wie ein Anker nach außen gebogen. Die Ankerform vermeidet zusätzliche an der Elektrode anzubringende Ankerteile und ist somit eine bevorzugte Ausführungsform. Es ist jedoch auch denkbar andere gebräuchliche Verankerungsformen zu wählen wie z.B. zickzack-Formen, V-Formen, usw.
Der differente Anker-Pol trägt zunächst die Herznadel, die an geeigneter Stelle im Myokard eingeführt wird und nach Verankerung des Pols abgeschnitten wird.

Leiter (1) und Leiter (2) können in üblicher Weise durch den Thorax nach außen geführt werden und an einen Herzschrittmacher oder EKG Monitor angeschlossen werden. Die Unterscheidbarkeit von Leiter (1) und (2) kann z.B. durch verschiedenfarbige Isolierungen erreicht werden.

Leiter (1) und (2) bestehen beispielsweise aus mindestens zwei isolierten Litzen.Die äußere Isolierung der Leiter (1) und (2) besteht aus einem biokompatiblen Kunststoff wie beispielsweise aus Silikon, Polyethylen oder Polyurethan.

Leiter (1) und (2)werden durch thermische Behandlung oder durch Verkleben der äußeren Isolierung miteinander verbunden.

Leiter (1) und (2) bestehen aus einem biokompatiblem leitenden Werkstoff wie Gold, Platin oder Platin Iridium, Edelstahl oder MP 35N, Nitinol, elektrisch leitfähige Fäden, Karbonfäden, elektrisch leitfähiger Kunststoff, auch mit Nanopartikeln gemischt.

Bei Implantation ist darauf zu achten, dass die Metallhülse (Anodenpol) ausserhalb der Herzoberfläche zu liegen kommt und somit nicht in das Herzgewebe hineingezogen wird.

Nachstehend ist die Erfindung anhand der Zeichnung noch näher beschrieben. Es zeigt in schematisierter Darstellung:
Fig. 1 eine gebräuchliche, im Handel erhältliche bipolare Elektrode.
Fig. 2 ein Ausschnitt der gebräuchlichen Elektrode von Fig. 1
Fig. 3 ein Ausführungsbeispiel der erfindungsgemäßen bipolaren Elektrode mit Metallhülse.
Fig. 4 ein Ausführungsbeispiel der erfindungsgemäßen bipolaren Elektrode mit blanker Litze.

**Fig. 1** zeigt eine gebräuchliche, im Handel erhältliche bipolare Elektrode. Die Elektrode hat im am Myokard zu fixierenden Bereich zwei mit Abstand zueinander angeordnete Pole (3) und (4). Pol (3) ist als Metallhülse ausgebildet und bildet den indifferenten Pol (Anode). Pol (4) ist der kathodische differente Anker Pol. Zwei gegeneinander isolierte Litzen bilden zwei Leiter (1) und (2). Die Metallhülse (3) umschließt beide Leiter. Leiter (1) wird bis zur Metallhülse (3) geführt und mit dieser elektrisch leitend verbunden. Leiter (2) durchläuft die Metallhülse (3) isoliert. Die Elektrode hat für ihre Anbringung am Myokard am distalen Ende eine gebogene chirurgische Nadel (5), welche nach dem Einsetzen der Elektrode abgetrennt wird. Ferner hat sie ein durch den Thorax nach außen zu führendes und in Gebrauchsstellung nach außen ragendes Ende (6).

**Fig. 2** ist ein Ausschnitt aus Fig 1 im Bereich der Pole (3) und (4).

**Fig. 3** zeigt die erfindungsgemäße bipolare Elektrode mit Metallhülse. Die Leiter (1) und (2) sind miteinander verbunden. Das geschieht z. B. durch thermische Behandlung. Leiter (1) ist distal mit einer Metallhülse (3) leitend verbunden, die den indifferenten Pol darstellt. Der isolierte Leiter (2) wird weitergeführt und bildet nach Ankerformung den differenten Pol (4). Dahinter befindet sich die später abtrennbare chirurgische Nadel (5) wie in Fig. 1 dargestellt.

**Fig. 4** zeigt die erfindungsgemäße bipolare Elektrode mit blankem indifferentem Pol. Die Leiter (1) und (2) sind wie in Fig. 2 beschrieben miteinander verbunden. Leiter (1) ist distal abisoliert (7). Die Isolation ist im distalen Bereich von Leiter (1) entfernt, sodass eine blanke Litze entsteht, die den indifferenten Pol (3) darstellt. Der isolierte Leiter (2) wird weitergeführt und bildet nach Ankerformung den differenten Pol (4). Dahinter befindet sich die später abtrennbare chirurgische Nadel wie in Fig. 1 dargestellt.

## Patentansprüche

1. Temporäre bipolare myokardiale Elektrode bestehend aus
zwei gegeneinander isolierten Leitern (1) und (2) mit je einem distalen und einem proximalen Ende, wobei Leiter (1) und (2) in Gebrauchsstellung aus dem Thorax nach außen ragen und mit ihrem proximalen Ende mit einem Herzschrittmacher oder EKG-Monitor verbunden werden und
einem ersten proximalen indifferenten Pol (3) und einem zweiten distalen differenten Pol (4) die mit Abstand zueinander angeordnet sind und in Gebrauchsstellung an der Herzwand verankert sind,
**dadurch gekennzeichnet, dass** die beiden gegeneinander isolierten Leiter (1) und (2) miteinander verbunden sind und gemeinsam bis zum proximalen indifferenten Pol (3) geführt werden, wobei Leiter (1) distal den indifferenten Pol (3) trägt und Leiter (2) isoliert außen am indifferenten Pol vorbeigeführt wird und distal den differenten Pol (4) trägt und dahinter zunächst die später abtrennbare chirurgische Nadel aufweist.

2. Elektrode gemäß Anspruch 1, wobei die isolierten Leiter (1) und (2) durch thermische Behandlung oder durch Verkleben der äußeren Isolierung miteinander verbunden sind.

3. Elektrode gemäß Anspruch 1, wobei der indifferente Pol (3) als Metallhülse ausgebildet ist.

4. Elektrode gemäß Anspruch 1, wobei der indifferente Pol (3) als blanke Litze ausgebildet ist..

5. Elektrode gemäß Anspruch 1, wobei der differente Pol (4) als Anker-Pol ausgebildet ist.
